# EUROPEAN PATENT SPECIFICATION

(11) **EP 2 346 412 B1**
(45) Date of publication and mention of the grant of the patent: **04.03.2015**
(21) Application number: 09776264.5
(22) Date of filing: 21.08.2009
(51) Int. Cl.: A61B 17/04, A61B 17/00

(54) **HIGH ASPECT RATIO SURGICAL ANCHOR AND METHOD OF USE**
CHIRURGISCHER ANKER MIT HOHEM ASPEKTVERHÄLTNIS UND ANWENDUNGSVERFAHREN
ANCRE CHIRURGICALE À RAPPORT DE FORME ÉLEVÉ ET PROCÉDÉ D'UTILISATION

(30) Priority: 29.08.2008 DK 200801200
(43) Date of publication of application: 27.07.2011
(73) Proprietor: Coloplast A/S, 3050 Humlebæk (DK)
(72) Inventor: WITZMANN, Michael, M., Minneapolis Minnesota 55418 (US); CLOOSE, Jeffrey, Evansville Minnesota 56326 (US); TRIEL, Egon, 3250 Gilleleje (DK)
(86) International application number: PCT/DK2009/050207
(87) International publication number: WO 2010/022729

(56) References cited:
- WO-A-01/39671
- WO-A-98/40114
- WO-A-03/077772
- WO-A-2008/057261

## Description

### FIELD OF THE INVENTION

The present invention relates generally to surgical devices and methods. The invention relates specifically to a high aspect ratio surgical anchor and its method of manufacture.

### BACKGROUND OF THE INVENTION

Urinary incontinence (UI) can range in severity from partial to complete loss of bladder control, and patients afflicted with UI can experience varying degrees of unintentional urine leakage. Incontinence conditions may change over time; and patients with light incontinence, for example, may experience minimal leakage during the occurrence of a provocative event, such as laughing or coughing, while those with heavy incontinence may experience continuous urine leakage.

Generally, UI is not considered a disease, but rather a symptom or side effect of another medical condition. Some causes of UI include prostate surgery and in particular total prostatectomies (in men), head and spinal cord injuries, infections, certain toxins such as excessive alcohol consumption, certain medications such as sedatives, and certain diseases such as cancer, Parkinson's disease, and multiple sclerosis. Indeed, incontinence can be caused simply by virtue of aging processes or even emotional distress.

As known to those in the urologic arts, there may be several types of UI and various ways to treat them. Stress urinary incontinence, which is a common type of incontinence, may be characterized as urine leakage during a provocative event such as sneezing, laughing, lifting heavy objects, or when the patient engages in any type of exercise that puts pressure on the bladder. Urge urinary incontinence occurs when the patient wants to urinate but is incapable of exercising restraint until reaching a restroom. Additional types of incontinence may include overflow incontinence, which occurs when the quantity of urine exceeds the capacity of the patient's bladder, and functional incontinence, which occurs when the patient has knowledge of the need to urinate but simply cannot access a restroom quickly enough due to a physical obstruction or debilitation.

To treat UI, several options may be available. These treatments may include behavioral techniques, such as biofeedback, bladder training, and pelvic muscle exercises; and modifications of the patient's diet and fluid intake. With respect to the latter, it is known that eliminating or reducing intake of certain types of substances, such as caffeine and alcohol, may help to alleviate incontinence. There are, additionally, medications available such as dicyclomine (Bentyl), flavoxate (Urispas), hyoscyamine sulfate (Anaspaz), imipramine (Tofranil), oxybutynin (Ditropan), tolterodine (Detrol), and propantheline (Pro-Banthine), phenylpropanolamine (Dexatrim), and pseudoephedrine (Sudafed), that may be helpful in controlling UI.

Surgery may additionally be an option to treat UI. Examples of commercially available surgical devices include suburethral slings for treatment of male UI such as the "InVance" device produced by American Medical Systems, Inc. (AMS) of Minneapolis, Minnesota. The "InVance" suburethral sling device is intended to provide structural support to the urethra for the treatment of stress urinary incontinence, thereby acting to retain urine within the bladder and not permit leakage through the urethra. Such "first generation" suburethral slings may not be easily used by urologists of ordinary skill in the art. For example, there may be problems with patients not being fully continent postoperatively due to the slings being anatomically fixated too loosely. Alternatively, there may also be problems with patients being put into retention postoperatively due to the devices being anatomically fixated too tightly.

Several companies have made attempts to progress the art of suburethral slings and their methods of use - in particular for treatment of female UI - by providing so-called "minimally invasive" slings for female patients. One such company is Johnson & Johnson (J&J) which developed a sling known as "TVT-Secur" (TVT-S). It may be surgically implanted using only a single vaginal incision. The sling is approximately 8 cm in length and has fixation tips fabricated from polyglactin 910 and polydioxanone suture yarn. The fixation tips of a TVT-S device may act to secure the sling until tissue in-growth may occur along the length of the sling. To implant a TVT-S sling, a standard vaginal dissection and two small paraurethral dissections are performed. The sling has pre-attached introducers that assist in placement to near a desired location within the two previously performed dissections. Once near the desired location, the introducer is detached from the sling and the other side of the sling is placed. The sling can be placed in a "U" or "hammock" configuration. The incision is then closed in standard fashion. Another company which provides a "minimally invasive" female suburethral sling is the aforementioned AMS. Its "Mini-Arc" suburethral sling device is approximately 8 cm in length with barb-like elements at each end. The barb-like elements may hold the sling in place until tissue in-growth may occur. Like the TVT-S device, a standard vaginal dissection and two small paraurethral dissections are performed to place the sling. A trocar-like instrument attaches to each barb-like element and assists in placement near a correct tissue plane. Once placed, the trocar releases from the barb-like element; placement then is made in the patient's contralateral side. The incision is then closed in standard fashion.

As with the aforementioned incontinence treatments for male patients, problems may also occur with use of "minimally invasive" slings for female patients. For example, the J&J TVT-S devices may experience several problems. The aforementioned two pre-attached introducers have rather bulky and sharp elements, which may cause excessive bleeding and hematomas - thereby making a procedure styled as "minimally invasive" in fact more invasive due to such complications. Also, fixation methods of TVT-S devices may be inadequate in that physicians may be unable to achieve continence in their patients due to faulty anchoring of the aforementioned fixation tips. The AMS "Mini-Arc" devices may function better than the J&J TVT-S devices; however, there may be two main drawbacks to these devices. First, the "Mini-Arc" device is relatively short which may impede a physician's ability to aim for a bony landmark as desired during implantation surgery. Desired repeatability of the surgical procedure from patient to patient is thereby diminished since anatomical landmarks may not be repeatably targeted, and instead the physician must rely on experience and subjective judgment to place the barb-like elements near correct tissue planes. Second, anchoring and tensioning functions in the "Mini-Arc" device may be coupled. That is, in placing a second anchor, the physician might need to correctly anticipate that the desired anchor placement location will ultimately result in desired tensioning of the sling.

Regardless of construction, mode of operation, or invasiveness, various examples of suburethral sling devices, components, and methods of manufacture and use, are described in (a) U.S. Pat. Nos. 6,626,916 (serial no. 09/445,011), 6,960,160 (serial no. 10/398,992), 7,229,404 (serial no. 10/524,861), 7,285,086 (serial no. 11/190,295), and 7,297,102 (serial no. 11/190,601); (b) U.S. Pat. Applic. Pub. Nos. 2005/0043820 (serial no. 10/492,473), 2006/0030884 (serial no. 10/914,059), 2006/0041185 (serial no. 11/199,601), 2006/0058578 (serial no. 10/510,488), 2006/0205995 (serial no. 11/324,028), 2007/0162120 (serial no. 11/615,144), and 2007/0299300 (serial no. 11/854,049); and (c) PCT Pat. Applic. Pub. Nos. WO 2007/097994 (serial no. PCT/US07/004015), WO 2007/149348 (serial no. PCT/US07/014120), WO 2007/149555 (serial no. PCT/US07/014553), and WO 2007/149593 (serial no. PCT/US07/014780).

WO 2008/057261 describes an implant, insertion tool, combinations, and associated methods, that involve placement of a self-fixating tip at tissue of the pelvic region, wherein an insertion tool includes one or more of an aperture for engaging a guide and an extension guard, the method optionally allowing for initial placement of a self-fixating tip at tissue of the pelvic region and adjustment of the location of the self-fixating tip. This document discloses the features defined in the preamble of claim 1. WO 2001/39671 describes a method of closing a tissue wound includes providing a wound closure device having a first anchor, a second anchor, and a flexible member movably attached to the second anchor, positioning the first anchor against tissue, passing the flexible member across the wound, positioning the second anchor against tissue, and pulling on a free end of the flexible member to shorten a length of the flexible member between the first and second anchors, thereby closing the wound.

WO 2003/077772 describes an elastically curved suture anchor is resiliently straightened and delivered into tissue by a needle. When the needle is withdrawn, resumption of the curvature provides leverage for anchor rotation as the attached suture is pulled to fasten the anchor within the tissue.

WO 98/40114 describes a template for guiding at least one suture through the periurethral fascia and vaginal mucosa adjacent a patient's urethra during a urethropexy procedure, wherein the at least one suture is attached to an anchor secured within the body of the patient above the patient's urethra, is provided. The template comprises first and second wing members extending laterally from opposite sides of the template; and at least one suture guide aperture positioned in each of the wing members at a predetermined location; the template configured to be alignable within the vagina of a patient such that one of the wing members will be positioned adjacent either side of the urethra with the at least one guide aperture in each wing member positioned such that a suture may be retrieved from within the patient's body through the at least one aperture.

In general, several drawbacks may be experienced in utilizing suburethral slings *per* se to treat UI. For example, securing suburethral sling devices into position may require use of known anchoring devices which could be difficult and time consuming to deploy, and which could result in unreliable anatomical fixation and unacceptably low pull-out forces. In addition, surgical procedures and methods for implanting suburethral sling devices for treatment of UI utilizing known anchoring devices may lead to difficulties in positioning and fixation with desired anatomical tensioning. Further, implantation of suburethral sling devices may be complex and time consuming, and may produce suboptimal clinical outcomes.

### SUMMARY OF THE INVENTION

In accordance with basic aspects of the present invention, a high aspect ratio surgical anchor could comprise a body defined in part along a major longitudinal axis, a fin coupled to the body, and a channel in the body along the longitudinal axis. The channel could be configured to receive and removably secure a tip of an introducer therewithin. A slot is provided through the body that is generally parallel to the longitudinal axis; and the slot could be configured to receive and pass implantable material therethrough and to secure a portion of the implantable material therein.

Also in accordance with basic aspects of the present invention, a method of manufacturing a high aspect ratio surgical anchor could comprise steps of making a mold in a shape of a high aspect ratio surgical anchor, filling the mold with a selected semi-liquid material and curing it, and then removing the anchor thereby created from the mold.

### BRIEF DESCRIPTION OF THE DRAWINGS

Figure 1 is a side view of an example of a high aspect ratio surgical anchor of the present invention.
Figure 1a is a top view of the high aspect ratio surgical anchor shown in figure 1.
Figure 1b is a bottom view of the high aspect ratio surgical anchor shown in figure 1.
Figure 2 is an illustration of the high aspect ratio surgical anchor shown in figures 1-1b, depicted as being in use during surgery.
Figure 3 is an illustration of the high aspect ratio surgical anchor shown in figures 1-2, depicted as being in use during surgery.
Figure 4 is an illustration of the high aspect ratio surgical anchor shown in figures 1-3, depicted as being in use during surgery.
Figure 5 is an illustration of the high aspect ratio surgical anchor shown in figures 1-4, depicted as being in use during surgery.
Figure 6 is an illustration of the high aspect ratio surgical anchor shown in figures 1-5, depicted as being in use during surgery.

### DETAILED DESCRIPTION OF THE INVENTION

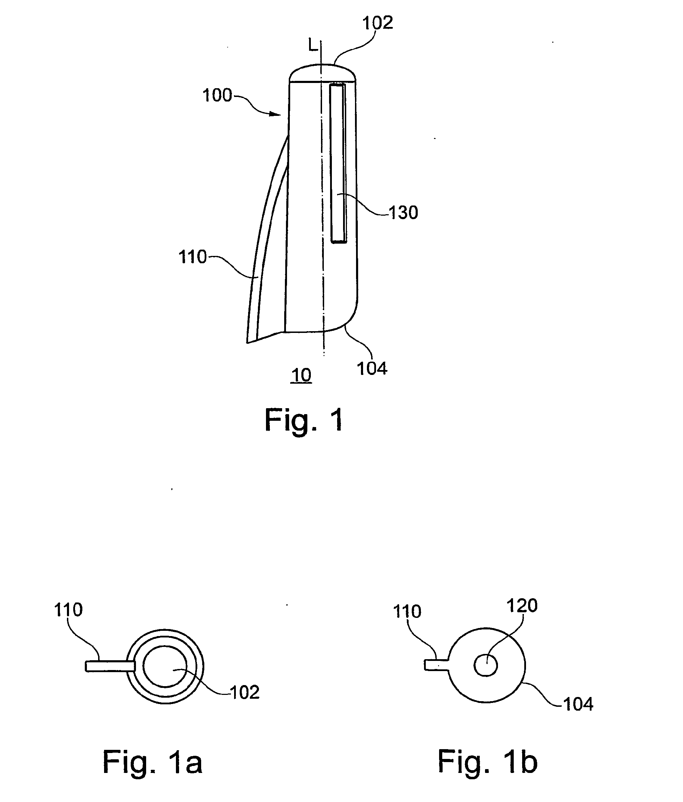

Illustrated in figures 1, 1a, and 1b is an example of a high aspect ratio surgical anchor 10 (hereinafter, "anchor 10") of the present invention. In this example, anchor 10 could include a body 100 defined in part along a major longitudinal axis L with a top portion 102 at one end of body 100 and a bottom portion 104 at an opposite end of body 100 along longitudinal axis L. A fin 110 could be coupled to, or otherwise formed integrally with, body 100. Fin 110 is intentionally provided to give anchor 10 a non-symmetrical profile as further described below. As particularly shown in fig. 1b, a channel 120 having its entrance in bottom portion 104 could be provided in body 100 along axis L. As will be further described in use of anchor 10, channel 120 could be configured to receive and removably secure a tip of an introducer therewithin. Also, as shown in fig. 1, a slot 130 could be provided through body 100 that is generally parallel to axis L. As will also be further described in use of anchor 10, slot 130 could be configured to receive and pass implantable material therethrough and to secure a portion of the implantable material therein. Dimensions of anchor 10 could be, for example, 0.320 inches (0.8128 cm) in length by 0.125 inches (0.3175 cm) in diameter, while channel 120 could be 0.045 inches (0.1143 cm) to 0.050 inches (0.1270 cm) in diameter and slot 130 could be 0.110 inches (0.2794 cm) in length by 0.020 inches (0.0508 cm) in width. Anchor 10 could be constructed using any suitable techniques as will be described below.

It is to be appreciated that, as shown particularly in the drawings, a high aspect ratio surgical anchor of the present invention intentionally embodies a relatively high "aspect ratio" or non-symmetrical profile as opposed to generally low aspect ratios or symmetrical profiles of heretofore known devices. In this regard, a relatively high aspect ratio could be characterized as a length to width ratio being greater than one. Thus, a greater difference or "spread" between length and width (i.e., difference between numerator and denominator in the ratio) results in a greater aspect ratio. A purpose of such an intentionally high aspect ratio profile will be appreciated with respect to use of the anchor of the present invention as will be described below.

Although not illustrated, it is to be understood that a high aspect ratio surgical anchor of the present invention, as exemplified by anchor 10, could be manufactured using any suitable materials (e.g., semi-liquid materials and metals such as stainless steel and Nitinol) and fabrication techniques. For example, a mold could be made in a shape of anchor 10, most notably including body 100, fin 110, channel 120, and slot 130. The mold could be filled with a selected semi-liquid material. The semi-liquid material could then be cured or cooled (hereinafter, individually or collectively, referred to as "cured" or "curing") and anchor 10 thereby created could then be removed from the mold. Alternatively, a mold could be made in a solid shape of body 100, whereupon channel 120 and slot 130 could be formed by any suitable technique such as by machining, drilling, or milling processes. The semi-liquid material could be, as desired or suitable for a particular manufacturing process, any suitable medical grade thermoplastic/thermoset and/or polymer-like material, of desired durometer for desired hardness, such as, for example, polyolefin, polypropylene, polyethylene, polyurethane, polycarbonate, polysulfone, so-called "ABS" polymer material, or nylon material; or combinations of these materials. Curing (or "cycle") times and temperatures, along with a particular selection of the material or materials, could determine optimal characteristics of thickness, hardness, and durability of body 100 and/or fin 110 of anchor 10. It is to be appreciated that, although again not illustrated herein, example anchor 10 could also be manufactured using any suitable fabrication techniques such as transfer molding or injection molding.

With reference now to all of the drawings, and in order to enable the skilled person to carry out the invention, a way of using a high aspect ratio surgical anchor of the present invention in treatment of urinary incontinence in a female patient is now described.

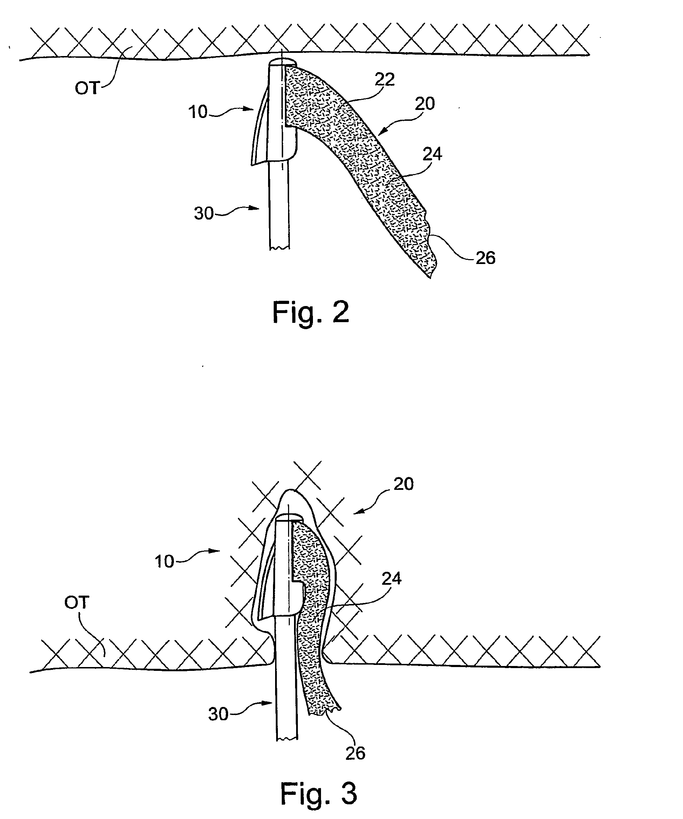

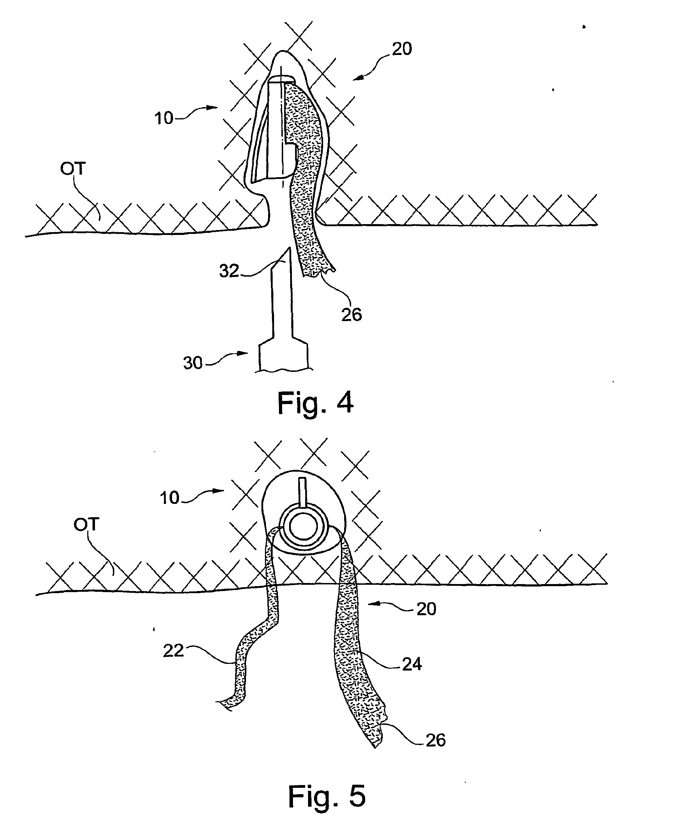

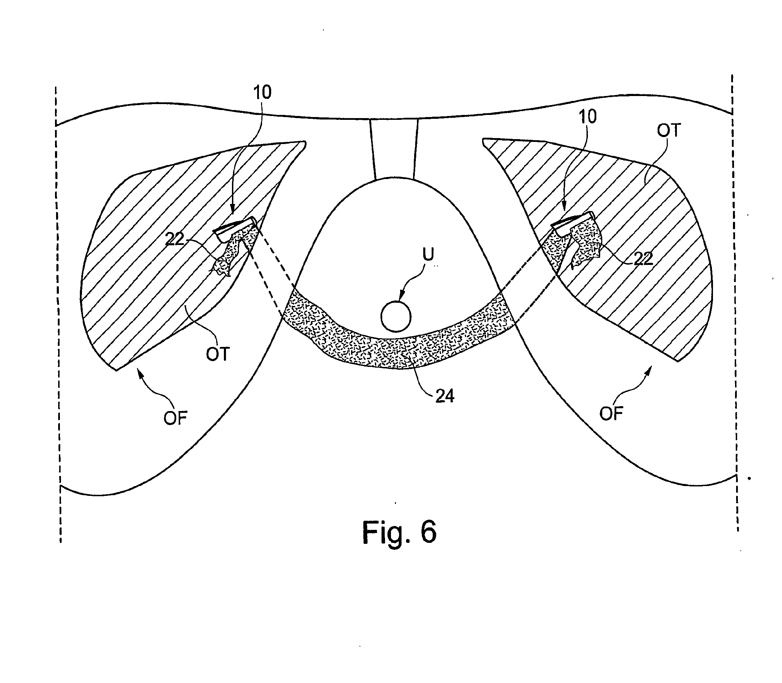

First and second high aspect ratio surgical anchors 10 could be provided to a physician, along with a suitable implantable material 20 and a suitable introducer 30. Implantable material 20 could have a first end portion 22, a center portion 24, and a second end portion 26. Material 20 could have a length of about 4 cm and a width of about 11mm. Suitable implantable material and a suitable introducer could be, for example, ARIS ® brand suburethral sling material and an ARIS ® brand curved introducer that are both commercially available from Coloplast A/S. The physician could then make vaginal dissection (or "midline dissection"; not illustrated) in the female patient to access internal anatomical space near the patient's obturator foramen OF (as shown in fig. 6). Referring to fig. 2, a selected portion of first end portion 22 of material 20 could then be placed through slot 130 of first anchor 10 such that material 20 is secured within slot 130. As further shown in fig. 2, introducer 30 could be removably coupled to anchor 10 by way of pushing a tip 32 of introducer 30 (as shown in fig. 4) into channel 120 of anchor 10 as shown in fig. 1b. Introducer 30, with anchor 10 coupled to it and material 20 in turn secured through slot 130 of anchor 10, could then be placed through the vaginal dissection so that introducer 30, anchor 10, and material 20 could all enter the internal anatomical space near the patient's obturator foramen OF. Introducer 30 with anchor 10 and material 20 could then be advanced into and through obturator tissue OT in the obturator foramen OF adjacent to a posterior side of an ischiopubic ramus. Commonly, an audible and/or tactile "pop" is noticed when obturator tissue is so penetrated. Introducer 30 could then be removed by pulling tip 32 outwardly from channel 120 in anchor 10 while major longitudinal axis L is generally perpendicular to a plane of obturator tissue OT as particularly shown in figs. 3 and 4; and subsequently introducer 30 could be withdrawn from the internal anatomical space and the vaginal dissection in the patient. The physician could then pull on end portion 26 of material 20 in the internal anatomical space to enable anchor 10 to essentially and intentionally "fall over" such that its major longitudinal axis L is generally parallel to the aforementioned plane of obturator tissue OT as particularly shown in figs. 5 and 6. In other words, anchor 10 thus has a high aspect ratio relative to penetrated obturator tissue OT as aforementioned. It is to be appreciated that, also as aforementioned, this "falling over" effect is provided by fin 110 that makes anchor 10 intentionally non-symmetrical in configuration and thus likely to "fall over" after tissue is penetrated. It is also to be understood that the intentional "falling over" of anchor 10 thus prevents anchor 10 from being able to be readily pulled back through penetrated obturator tissue OT, thereby effectively anchoring material 20 in obturator foramen OF. The aforedescribed steps could then be repeated on the patient's contralateral side with respect to placement of second anchor 10 as particularly illustrated in fig. 6. Center portion 24 of material 20 could then be manipulated by the physician through the vaginal dissection to ensure placement of center portion 24 near a urethra U of the patient so that urethra U could be supported or even compressed as may be necessary for a particular UI treatment. Optionally, the physician could also pull on material 20 through the dissection to achieve desired tensioning of material 20 relative to urethra U. Finally, the vaginal dissection could be closed by any suitable surgical technique such as suturing, thereby concluding the implantation surgery.

It is to be appreciated that although this example of a surgical way of utilizing anchor 10 has been described relative to female patients, it could of course also be performed in male patients with corresponding and/or alternative surgical steps (e.g., a perineal incision in place of a vaginal dissection).

It is to be also appreciated that the aforedescribed example of a way of utilizing the high aspect ratio surgical anchor of the present invention is intended to be a "minimally invasive" treatment for stress UI. It is to be understood, with particular reference to fig. 6, that material 20 could "cradle" the patient's urethra U. Material 20 could be effectively fixed in one obturator foramen OF, via one anchor 10 that has penetrated obturator tissue OT, and span the patient's internal anatomical space, under urethra U, to the contralateral obturator foramen OF whereupon material 20 could be similarly fixed in the contralateral obturator foramen OF via another anchor 10 that has penetrated obturator tissue OT as aforedescribed. Thus, material 20 could effectively reside between urethra U and the patient's anterior vaginal wall adjacent to the vaginal dissection performed by the physician. This "cradle" of material 20 could serve to support urethra U in response to a stress event such as coughing or laughing, thereby preventing stress UI. Material 20 could have an ability, in this example, to be tensioned by pulling on an end 22 of material 20 that passes through anchor 10. As more material 20 is passed through anchor 10, an amount of material 20 between both obturator foramen OF decreases which thereby could provide desired tension against or lifting force under urethra U. It has been discovered that a physical combination of material 20 passing through anchor 10 and material 20 passing through obturator tissue OT could provide resistance to unintentional loosening of material 20 while tensioning thereof is being performed as aformentioned. It has also been discovered that upon occurrence of so-called "tissue in-growth" of material 20 after the surgery is completed and during the patient's healing process, anchors 10 may no longer be actually needed to fix material 20 in the patient; and therefore anchors 10 could be made of a suitable medical grade bioresorbable material.

It is to be also appreciated that the foregoing example of a way to treat UI provides an anchor placement step that, notably unlike the AMS "Mini-Arc" device, is intentionally "decoupled" from tensioning of the suburethral sling material. In the foregoing example, both anchors could be placed and checked for desired positioning. Thereafter, when the anchors are in desired positions, tensioning of the sling material could then be performed. In the "Mini-Arc" device, however, tensioning is in fact "coupled" to anchor fixation - distal or proximal placement of a second anchor with respect to the urethra dictates the tension of the sling material.

It is to be additionally appreciated that the foregoing example of a way to treat UI could provide an ability for the physician to place the anchors near discrete "bony landmarks". This ability could be another distinguishing factor from the AMS "Mini-Arc" device, which intentionally utilizes a relative short length of sling material and thereby impedes a physician from placing anchors near such "bony landmarks". Rather, a physician implanting an AMS device must find a suitable location for anchor placement by subjective reliance on past surgical experience.

It is to be further appreciated that the high aspect ratio surgical anchor of the present invention advantageously changes its configuration or profile upon anatomical placement. Specifically, the anchor advantageously presents a relatively small profile upon penetration of tissue. Then, upon "falling over", the anchor presents a relatively large profile and thus, potential trauma to the patient's anatomy may be thereby minimized. Other known anchoring means - such as in TVT-S, for example - may not have this advantage since their anchoring configurations or profiles remain relatively constant throughout implantation surgery.

Although not illustrated in the drawings, it is to be appreciated that a high aspect ratio surgical anchor provided in accordance with the present invention could have (i) a relatively pointed cap portion (corresponding, e.g., to top portion 102 in example anchor 10) to facilitate penetration of a patient's anatomical tissue such as the aforementioned obturator tissue, or (ii) a hole in the cap portion so that a tip of an introducer could protrude therefrom (e.g., through channel 120 in example anchor 10), or even a combination of these attributes (i) and (ii).

It is to be appreciated from the foregoing disclosure that the present invention uniquely and advantageously satisfies the long-felt need for a high aspect ratio surgical anchor that may be easier to surgically secure into position than known anchoring devices, and that may be simply constructed, manufactured at relatively low cost, and easy to use when compared to known anchoring devices.

It is to be understood that novel aspects of the present invention will be appreciated by those in the surgical arts to be capable of use in, and beneficial to, virtually any anatomical anchoring *per se -* even outside of UI treatment technologies.

While the present invention has been particularly shown and described with reference to the accompanying specification and drawings, it will be understood however that other modifications thereto are of course possible; and all of which are intended to be within the scope of the present invention. It should be appreciated that (i) components, dimensions, shapes, and other particulars of example embodiments of the invention aforedescribed may be substituted for others that are suitable for achieving desired results, (ii) various additions or deletions may be made thereto, and (iii) features of the foregoing examples may also be made in combinations thereof. It is also to be understood in general that any suitable alternatives may be employed to provide the high aspect ratio surgical anchor of the present invention, its manufacturing method.

Lastly, of course, the choice of compositions, sizes, and strengths of various aforementioned elements of the present invention are all a matter of design choice depending upon intended uses thereof.

Accordingly, these and other various changes or modifications in form and detail of the present invention may also be made therein, again without departing from the scope of the invention as defined by the appended claims.

## Claims

1. A high aspect ratio surgical anchor (10), comprising:
a body (100) defined in part along a major longitudinal axis (L);
a fin (110) coupled to said body (100);
a channel (120) in said body (100) along said longitudinal axis (L), said channel (120) being configured to receive and removably secure a tip (32) of an introducer (30) therewithin **characterized in that** the anchor further comprises
a slot (130) through said body (100) that is generally parallel to said major longitudinal axis (L), said slot (130) being configured to receive and pass implantable material therethrough and to secure a portion of said implantable material therein.

2. The high aspect ratio surgical anchor of claim 1, further comprising a pointed cap (102) portion in said body (100).

3. The high aspect ratio surgical anchor of claim 1, wherein said channel (120) passes fully through said body (100) so that a tip (32) of an introducer (30) placed therein can protrude from said body (100).

4. A method of manufacturing a high aspect ratio surgical anchor (10), comprising steps of:
making a mold in a shape of a high aspect ratio surgical anchor (10) of claim 1;
filling said mold with a selected semi-liquid material;
curing said semi-liquid material; and
removing said high aspect ratio surgical anchor (10) thereby created from said mold.

5. The method of manufacturing a high aspect ratio surgical anchor of claim 4, further comprising a technique selected from a group consisting of transfer molding and injection molding.

6. The method of manufacturing a high aspect ratio surgical anchor of claim 4, wherein said semi-liquid material is selected from a group consisting of a medical grade thermoplastic material, a medical grade thermoset material, a medical grade polymer-like material, and a medical grade bioresorbable material.

7. The method of manufacturing a high aspect ratio surgical anchor of claim 6, wherein said semi-liquid material is further selected from a group consisting of polyolefin, polypropylene, polyethylene, polyurethane, polycarbonate, polysulfone, so-called "ABS" polymer material, and nylon material.

## Patentansprüche

1. Chirurgischer Anker (10) mit hohem Aspektverhältnis, umfassend:
einen Körper (100), der teilweise entlang einer Hauptlängsachse (L) definiert ist;
eine mit dem Körper (100) verbundene Rippe (110);
einen Kanal (120) in dem Körper (100) entlang der Längsachse (L), wobei der Kanal (120) zur Aufnahme und lösbaren Fixierung einer Spitze (32) einer Einführungsvorrichtung (30) darin ausgelegt ist,
**dadurch gekennzeichnet, dass** der Anker ferner einen Schlitz (130) durch den Körper (100) umfasst, der allgemein parallel zu der Hauptlängsachse (L) verläuft, wobei der Schlitz (130) zur Aufnahme und Durchleitung von implantierbarem Material durch ihn hindurch und zur Fixierung eines Abschnitts des implantierbaren Materials darin ausgelegt ist.

2. Chirurgischer Anker mit hohem Aspektverhältnis nach Anspruch 1, ferner einen spitz zulaufenden Kappenabschnitt (102) in dem Körper (100) umfassend.

3. Chirurgischer Anker mit hohem Aspektverhältnis nach Anspruch 1, worin der Kanal (120) vollständig durch den Körper (100) verläuft, so dass eine darin platzierte Spitze (32) einer Einführungsvorrichtung (30) aus dem Körper (100) vorstehen kann.

4. Verfahren zur Herstellung eines chirurgischen Ankers (10) mit hohem Aspektverhältnis, das folgende Schritte umfasst:
Herstellung einer Form mit der Gestalt eines chirurgischen Ankers (10) mit hohem Aspektverhältnis nach Anspruch 1;
Füllen der Form mit einem ausgewählten halbflüssigen Material;
Aushärten des halbflüssigen Materials; und
Entnehmen des dadurch erzeugten chirurgischen Ankers (10) mit hohem Aspektverhältnis aus der Form.

5. Verfahren zur Herstellung eines chirurgischen Ankers mit hohem Aspektverhältnis nach Anspruch 4, ferner eine Technik umfassend, die aus einer Gruppe ausgewählt ist, die aus Transferpressen und Spritzgießen besteht.

6. Verfahren zur Herstellung eines chirurgischen Ankers mit hohem Aspektverhältnis nach Anspruch 4, worin das halbflüssige Material aus einer Gruppe ausgewählt ist, die aus einem medizinischen thermoplastischen Material, einem medizinischen duroplastischen Material, einem medizinischen polymerartigen Material und einem medizinischen bioresorbierbaren Material besteht.

7. Verfahren zur Herstellung eines chirurgischen Ankers mit hohem Aspektverhältnis nach Anspruch 6, worin das halbflüssige Material ferner aus einer Gruppe ausgewählt ist, die aus Polyolefin, Polypropylen, Polyethylen, Polyurethan, Polycarbonat, Polysufon, sogenanntem "ABS"-Polymermaterial und Nylonmaterial besteht.

## Revendications

1. Ancre chirurgicale à rapport de forme élevé (10), comprenant:
un corps (100) qui est défini en partie le long d'un axe longitudinal majeur (L);
une ailette (110) qui est couplée audit corps (100) ;
un canal (120) dans ledit corps (100) le long dudit axe longitudinal (L), ledit canal (120) étant configuré de manière à recevoir et à fixer de façon détachable une pointe (32) d'un introducteur (30) à l'intérieur de celui-ci,
**caractérisée en ce que** l'ancre comprend en outre:
une fente (130) à travers ledit corps (100) qui est essentiellement parallèle audit axe longitudinal majeur (L), ladite fente (130) étant configurée de manière à recevoir et à faire passer une matière implantable à travers celle-ci et à fixer une partie de ladite matière implantable dans celle-ci.

2. Ancre chirurgicale à rapport de forme élevé selon la revendication 1, comprenant en outre une partie de coiffe pointue (102) dans ledit corps (100).

3. Ancre chirurgicale à rapport de forme élevé selon la revendication 1, dans laquelle ledit canal (120) passe entièrement à travers ledit corps (100) de telle sorte qu'une pointe (32) d'un introducteur (30) placé dans celui-ci puisse faire saillie à partir dudit corps (100).

4. Procédé de fabrication d'une ancre chirurgicale à rapport de forme élevé (10), comprenant les étapes suivantes:
fabriquer un moule ayant la forme d'une ancre chirurgicale à rapport de forme élevé (10) selon la revendication 1;
remplir ledit moule avec une matière semi-liquide sélectionnée;
faire durcir ladite matière semi-liquide; et
enlever ladite ancre chirurgicale à rapport de forme élevé (10) ainsi créée hors dudit moule.

5. Procédé de fabrication d'une ancre chirurgicale à rapport de forme élevé selon la revendication 4, comprenant en outre une technique qui est sélectionnée dans un groupe comprenant le moulage par transfert et le moulage par injection.

6. Procédé de fabrication d'une ancre chirurgicale à rapport de forme élevé selon la revendication 4, dans lequel ladite matière semi-liquide est sélectionnée dans un groupe comprenant une matière thermoplastique de qualité médicale, une matière thermodurcissable de qualité médicale, une matière de type polymère de qualité médicale, et une matière biorésorbable de qualité médicale.

7. Procédé de fabrication d'une ancre chirurgicale à rapport de forme élevé selon la revendication 6, dans lequel ladite matière semi-liquide est en outre sélectionnée dans un groupe comprenant la polyoléfine, le polypropylène, le polyéthylène, le polyuréthane, le polycarbonate, le polysulfone, une matière polymère dite "ABS", et une matière de nylon.
